(19) Europäisches Patentamt
European Patent Office
Office européen des brevets

(11) **EP 4 170 088 A1**

(12) **EUROPÄISCHE PATENTANMELDUNG**

(43) Veröffentlichungstag:
26.04.2023 Patentblatt 2023/17

(21) Anmeldenummer: **21204272.5**

(22) Anmeldetag: **22.10.2021**

(51) Internationale Patentklassifikation (IPC):
***D07B 1/14*** *(2006.01)*

(52) Gemeinsame Patentklassifikation (CPC):
(C-Sets verfügbar)
**D07B 1/145; G01L 5/101;** A61B 34/30; A61B 34/71;
A61B 2090/064; D04C 1/12; D07B 2201/1096;
D07B 2201/209; D07B 2205/106; D07B 2205/2014;
D07B 2205/2042; D07B 2205/2046;
D07B 2205/205; D07B 2205/2096;
D07B 2205/3067; (Forts.)

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Siemens Aktiengesellschaft**
**80333 München (DE)**

(72) Erfinder:
• **Mose, Christian**
**81476 München (DE)**
• **Freitag, Reinhard**
**82008 Unterhaching (DE)**
• **Bachmann, Christian**
**80686 München (DE)**
• **Nemoto, Takeru**
**80333 München (DE)**

(74) Vertreter: **Siemens Patent Attorneys**
**Postfach 22 16 34**
**80506 München (DE)**

(54) **ZUGSEIL, SEILBETRIEBENE EINRICHTUNG UND MESSVERFAHREN**

(57) Es wird ein Zugseil (1) zur Übertragung einer axialen Zugkraft ($F_A$) angegeben, umfassend
- einen schlauchförmigen Seilmantel (3), der ein Geflecht aus mehreren Seilsträngen und/oder Fasern (5) umfasst, wobei die einzelnen Seilstränge und/oder Fasern (5) schräg zur lokalen Seilachse (A) verlaufen, und
- ein in einem Teilabschnitt innerhalb des Seilmantels (3) angeordnetes länglich geformtes Sensorelement (11), welches ein verformbares längliches Widerstandselement (20) aufweist, dessen elektrischer Längswiderstand von einem radialen Druck ($p_r$) des Seilmantels (3) auf das Sensorelement (11) abhängt,
- wobei der radiale Druck ($p_r$) auf das Sensorelement (11) von der axialen Zugkraft ($F_A$) auf das Zugseil (1) abhängt. Weiterhin wird eine seilbetriebene Einrichtung mit einem solchen Zugseil (1) angegeben sowie ein Verfahren zur Messung einer Zugkraft ($F_A$), welche auf ein solches Zugseil (1) einwirkt.

FIG 6

(52) Gemeinsame Patentklassifikation (CPC): (Forts.)
D07B 2301/258; D07B 2301/355;
D07B 2301/3566; D07B 2301/554;
D07B 2501/2069

C-Sets
D07B 2205/106, D07B 2801/10, D07B 2801/22;
D07B 2205/2014, D07B 2801/10, D07B 2801/22;
D07B 2205/2042, D07B 2801/10, D07B 2801/22;
D07B 2205/2046, D07B 2801/10, D07B 2801/22;
D07B 2205/205, D07B 2801/10, D07B 2801/22;
D07B 2205/2096, D07B 2801/10, D07B 2801/22;
D07B 2205/3067, D07B 2801/10, D07B 2801/22

## Beschreibung

Die vorliegende Erfindung betrifft ein Zugseil zur Übertragung einer axialen Zugkraft. Dieses Zugseil weist einen schlauchförmigen Seilmantel auf, der ein Geflecht aus mehreren Seilsträngen und/oder Fasern umfasst, wobei die einzelnen Seilstränge und/oder Fasern schräg zur lokalen Seilachse verlaufen. Weiterhin betrifft die Erfindung eine seilbetriebene Einrichtung mit einem solchen Zugseil und ein Verfahren zur Messung einer Zugkraft auf einem Zugseil.

Aus dem Stand der Technik sind hochfeste Seile bekannt, welche zum Übertragen von Zugkräften in seilbetriebenen Einrichtungen dienen. So werden beispielsweise in Robotikeinrichtungen Zugseile eingesetzt, welche ähnlich wie die Sehnen im menschlichen Körper der Kraftübertragung zwischen einer Antriebseinheit und einem davon entfernten Abtriebselement dienen. Es existieren aber auch zahlreiche andere Anwendungen im Maschinenbau und in der Elektrotechnik, bei der kraftübertragende Seilkinematiken eingesetzt werden. Durch die Entwicklung von hochfesten Kunststoffen als Seilmaterialien können solche Seilkinematiken zunehmend kompakter ausgestaltet werden. Für viele dieser Anwendungen ist es nützlich, den aktuellen Belastungszustand des verwendeten Zugseils zu kennen und insbesondere kontinuierlich zu messen. Prinzipiell sind im Stand der Technik Methoden zur Zugspannungsmessung an Seilen bekannt. So werden verschiedene Handgeräte und auch stationäre Zugspannungs-Messgeräte kommerziell angeboten, bei welchen ein unter Zugspannung stehendes Seil typischerweise zwischen mehreren Rollen eingespannt wird, wobei die auf die Rollen einwirkenden Kräfte mit Kraftsensoren gemessen werden. Nachteilig an dieser Methode ist, dass die Messung aufgrund der zusätzlichen Umlenkrollen eine Änderung der Seilführung erfordert. Außerdem wird für diese Art der Messung typischerweise relativ viel Bauraum benötigt, da ein hinreichend langer freier Seilabschnitt vorliegen muss, um das Messgerät daran anzuordnen. Ein derartig langer freier Seilabschnitt ist aber gerade in kompakten Robotikeinrichtungen und anderen kompakten Seilkinematiken nicht immer vorhanden bzw. nicht in allen Betriebszuständen zugänglich. Außerdem muss in wenigstens ein Umlenk-Element ein Kraftsensor eingebaut werden.

Aufgabe der Erfindung ist es, ein Zugseil zur Verfügung zu stellen, welches die genannten Nachteile überwindet. Insbesondere soll ein Zugseil zur Verfügung gestellt werden, welches während seines Betriebs in einer seilbetriebenen Einrichtung auf einfache und platzsparende Weise eine Messung der einwirkenden Zugkraft ermöglicht. Weiterhin soll durch die Messanordnung vorteilhaft die Funktionalität des Zugseils und insbesondere dessen Flexibilität und/oder dessen Zugfestigkeit möglichst wenig beeinflusst werden. Außerdem soll die Messung möglichst kontinuierlich, während des gesamten Betriebs möglich sein. Eine weitere Aufgabe ist es, eine seilbetriebene Einrichtung mit einem solchen Zugseil und ein Verfahren zur Messung der Zugkraft an einem solchen Zugseil zur Verfügung zu stellen.

Diese Aufgaben werden durch das in Anspruch 1 beschriebene Zugseil, die in Anspruch 12 beschriebene seilbetriebene Einrichtung und das in Anspruch 14 beschriebene Messverfahren gelöst.

Das erfindungsgemäße Zugseil ist zur Übertragung einer axialen Zugkraft ausgelegt. Es umfasst einen schlauchförmigen Seilmantel, der ein Geflecht aus mehreren Seilsträngen und/oder Fasern aufweist, wobei die einzelnen Seilstränge und/oder Fasern schräg zur lokalen Seilachse verlaufen. Erfindungsgemäß ist in einem Teilabschnitt innerhalb des Seilmantels ein länglich geformtes Sensorelement angeordnet. Dieses Sensorelement weist ein verformbares längliches Widerstandselement auf, dessen elektrischer Längswiderstand von einem radialen Druck des Seilmantels auf das Sensorelement abhängt. Dabei hängt der radiale Druck auf das Sensorelement von der axialen Zugkraft auf das Zugseil ab.

Die Bezeichnungen "axial" und "radial" beziehen sich hierbei jeweils auf die lokale Seilachse, also auf die Längsrichtung des Seils in dem betrachteten Seilabschnitt. Auch bei Zugseilen mit nicht kreisförmigem Querschnitt soll dabei der Begriff "radial" analog zur Kreisform die Richtung von einem außenliegenden Punkt zum Zentrum des Seils (oder umgekehrt) bedeuten. Der Seilmantel umfasst mehrere schräg zur Seilachse liegende Seilstränge und/oder Fasern, wie dies generell bei Seilen üblich ist. Beispielsweise werden Kletterseile heutzutage als Kern-Mantel-Seile gefertigt, bei denen ein außenliegender Seilmantel einen innenliegenden Seilkern radial umgibt. Der hier beschriebene Seilmantel entspricht dem Seilmantel eines solchen Kern-Mantel-Seils, wobei der innenliegende Seilkern prinzipiell (zumindest teilweise) vorhanden sein kann, aber besonders vorteilhaft auch ganz entfallen kann. Anstelle des Seilkerns oder ggf. auch zusätzlich zu diesem ist im Inneren des Seilmantels das länglich geformte Sensorelement angeordnet. Dieses Sensorelement ermöglicht eine Messung der Zugspannung *in situ,* nämlich im Zugseil selbst. Das Sensorelement ist dazu ausgelegt, einen radialen Druck des Seilmantels auf das Sensorelement zu messen. Dies wird dadurch ermöglicht, dass das Sensorelement ein reversibel verformbares Widerstandselement umfasst, dessen elektrischer Längswiderstand durch eine Änderung dieses radialen Drucks variiert werden kann. Der Aufbau des Seilmantels mit mehreren schräg verlaufenden Seilsträngen und/oder Fasern bewirkt außerdem, dass bei einer Änderung der axialen Zugkraft der radiale Druck des Seilmantels auf das Sensorelement verändert wird. Dies geschieht dadurch, dass aufgrund der Schräglage zur Achse eine Zugkraft entlang der Seilachse immer ein Anpressen des Seilmantels in Richtung der Seilmitte bewirkt, wobei der Winkel der einzelnen Stränge bzw. Fasern mit der Seilachse kleiner wird. Umgekehrt führt ein Nachlassen der Zugkraft zu einer Reduktion der Spannung und dabei abhängig von der Einbausituation zu einer Verkürzung und gleichzeitigen Stauchung des Seilmantels, bei welcher sich der Winkel der Stränge oder Fasern zur Seilachse vergrößert, entsprechend der Durchmesser des Seilmantels vergrößert wird und der radiale Druck auf das längliche Sensorelement nachlässt. Die einzelnen Stränge bzw. Fasern können miteinander verstrickt, verwoben, verdreht, verschränkt und/oder verflochten sein. Wesentlich ist nur, dass zumindest ein überwiegender Teil der Fasern bzw. Stränge einen Winkel mit der Längsachse einschließt, so dass die beschriebene Wechselwirkung zwischen axialer Zugkraft und radialem Druck auf das innenliegende Sensorelement zustande kommt. Ein einzelner Seilstrang kann seinerseits aus mehreren Fasern verstrickt, verwoben, verdreht, verschränkt und/oder verflochten sein. Es können aber auch bereits einzelne Fasern aus Garn oder Draht in entsprechender Weise zu dem Seilmantel zusammengesetzt sein. Die längliche Form des innenliegenden Sensorelements ist zweckmäßig, um bei zunehmender Zugkraft eine Zentrierung des Sensorelements im Inneren des Seilmantels zu bewirken.

Ein wesentlicher Vorteil des erfindungsgemäßen Zugseils liegt darin, dass mit dem innenliegenden Sensorelement auf einfache Weise eine Messung der Zugspannung am Ort des Seils ermöglicht wird, ohne dass hierfür ein großer zusätzlicher Platzbedarf entsteht. Es muss lediglich ein relativ kurzer freier Seilabschnitt zur Verfügung gestellt werden, in dem das Sensorelement angeordnet werden kann. Beispielsweise kann die Länge dieses Teilabschnitts z.B. das 20fache des Seildurchmessers oder weniger betragen. Beispielsweise kann die Länge des Seilabschnitts zwischen dem 4fachen und dem 20fachen des Seildurchmessers betragen. Aufgrund der Integration des Sensors im Inneren des Seils wird ein sehr kompakter Aufbau zur Messung der Zugspannung zur Verfügung gestellt, welcher sich insbesondere für die Verwendung in kompakten seilbetriebenen Einrichtungen eignet. Dadurch wird eine dauerhafte Überwachung der Zugspannung beim Betrieb des Zugseils ermöglicht. Die Ausgestaltung des Sensorelements mit einem abhängig vom radialen Druck variablen elektrischen Widerstand ermöglicht eine für viele Anwendungen ausreichend genaue Kraftmessung bei gleichzeitig apparativ einfachem und platzsparendem Aufbau. Die mechanischen Eigenschaften des Zugseils werden durch das integrierte Sensorelement nicht wesentlich verändert, insbesondere wenn der betreffende Seilabschnitt innerhalb einer seilbetriebenen Einrichtung in einem freien Bereich des Seils - also zwischen den dort vorliegenden Umlenk- bzw. Befestigungspunkten - angeordnet ist. Insbesondere werden die Zugfestigkeit des Seils und die Steifigkeit in axialer Richtung durch die innenliegende Anordnung des Sensorelements nur unwesentlich beeinflusst. So kann durch die Erfindung eine dauerhafte Integration einer Sensorik in das Zugseil zur kontinuierlichen Messung der Zugkraft ermöglicht werden.

Die erfindungsgemäße seilbetriebene Einrichtung weist eine Antriebseinheit, ein Abtriebselement und ein erfindungsgemäßes Zugseil auf, welches die Antriebseinheit und das Abtriebselement bezüglich einer Zugrichtung kraftübertragend verbindet. Die erfindungsgemäße Ausgestaltung des Zugseils ermöglicht auf einfache Weise eine Messung der Zugspannung innerhalb der Einrichtung, wobei für diese Funktionalität kaum zusätzlicher Bauraum benötigt wird. Die beschriebene Verbindung zwischen Antriebseinheit und Abtriebselement über das Zugseil muss keine direkte Verbindung sein. Mit anderen Worten sollen weitere verbindende Zwischenelemente nicht ausgeschlossen sein. Die kraftübertragende Verbindung soll aber über das Zugseil vermittelt sein.

Das erfindungsgemäße Verfahren dient zur Messung einer Zugkraft, welche auf ein erfindungsgemäßes Zugseil einwirkt. Dabei wird ein durch den Längswiderstand des reversibel verformbaren länglichen Widerstandselements beeinflusstes elektrisches Signal gemessen, dessen Größe von einem radialen Druck des Seilmantels auf das Sensorelement abhängt. Weiterhin hängt der radiale Druck auf das Sensorelement von der Zugkraft auf das Zugseil ab. Die Vorteile der erfindungsgemäßen seilbetriebenen Einrichtung und des erfindungsgemäßen Messverfahrens ergeben sich analog zu den oben beschriebenen Vorteilen des erfindungsgemäßen Zugseils.

Vorteilhafte Ausgestaltungen und Weiterbildungen der Erfindung gehen aus den von den Ansprüchen 1, 12 und 14 abhängigen Ansprüchen sowie der folgenden Beschreibung hervor. Dabei können die beschriebenen Ausgestaltungen des Zugseils, der seilbetriebenen Einrichtung und des Messverfahrens allgemein vorteilhaft miteinander kombiniert werden.

So kann das länglich geformte Sensorelement allgemein vorteilhaft mit seiner Längsachse entlang der lokalen Seilachse ausgerichtet sein und ein Aspektverhältnis von wenigstens 2:1 aufweisen. Unter diesem Aspektverhältnis soll das Verhältnis von Länge zu (größtem) Durchmesser des Sensorelement verstanden werden. Ein derartig ausgestaltetes Sensorelement wird durch die auf das Seil einwirkende Zugkraft im Inneren des Seilmantels zentriert.

Allgemein vorteilhaft und unabhängig von der genauen Formgebung können der Seilmantel und das innenliegende Sensorelement koaxial zueinander ausgestaltet sein. Besonders vorteilhaft weisen dabei sowohl der außenliegende Seilmantel als auch das innenliegende Sensorelement eine rotationssymmetrische Grundform auf, besitzen also jeweils eine kreisförmige Querschnittsgeometrie. Bei der koaxialen Anordnung dieser beiden insbesondere rotationssymmetrischen Formen liegt aufgrund der Symmetrie eine besonders geringe Beeinflussung der Eigenschaften des Seilmantels vor. Die genannte rotationssymmetrische Form von Seilmantel und Sensorelement ist jedoch nicht zwingend: So kann die Erfindung prinzipiell auch realisiert werden, wenn z.B. ein Sensorelement mit flacher Querschnittsform innerhalb eines entsprechend flach geformten Seilmantels angeordnet ist.

Der Winkel der einzelnen Seilstränge und/oder Fasern zur lokalen Seilachse kann vorteilhaft zwischen 15° und 40° liegen. Der genaue Winkel ist zwar abhängig von der im jeweiligen Betriebszustand einwirkenden Zugkraft, doch kann bei allen üblicherweise im Betrieb auftretenden Zugkräften der sogenannte Flechtwinkel im angegebenen Bereich liegen.

Gemäß einer allgemein vorteilhaften Ausführungsform weist das verformbare längliche Widerstandselement eine leitfähige Flüssigkeit oder leitfähige Paste als Leitermaterial auf. Besonders vorteilhaft kann es sich bei diesem flüssigen Leitermaterial um ein Flüssigmetall handeln. Generell sind flüssige Leitermaterialen besonders leicht reversibel verformbar und sind daher besonders gut geeignet, durch ihre Verformung aufgrund einer Änderung des radialen Drucks eine messbare Widerstandsänderung zu bewirken. Flüssigmetalle weisen neben diesen günstigen Verformungseigenschaften zudem eine vergleichsweise hohe Leitfähigkeit auf, so dass die durch eine Zugspannungsänderung verursachte geometrische Veränderung des Widerstandselements besonders einfach und vergleichsweise präzise durch ein elektrisches Signal messbar ist.

Unter einem Flüssigmetall soll hier allgemein ein Metall (also ein metallisches Element oder auch eine Legierung mehrerer metallischer Elemente) verstanden werden, welches bei einem Betriebszustand des Zugseils in einem flüssigen Aggregatzustand ist. Beispielsweise kann das Flüssigmetall bei Raumtemperatur in einem flüssigen Zustand vorliegen. Ein solches Flüssigmetall kann vorteilhaft Gallium, Indium, Zinn und/oder Quecksilber umfassen. Derartige Metalle sind vorteilhafte Komponenten, um in einer metallischen Legierung einen niedrigen Schmelzpunkt zu erreichen. Besonders vorteilhaft weist das flüssige Widerstandselement sowohl Gallium als auch Indium und Zinn auf. Gemäß einer besonders bevorzugten Ausführungsform besteht es sogar ausschließlich aus den drei genannten Metallen. Beispielsweise kann es sich bei dem Flüssigmetall um eine Legierung handeln, die in der Fachwelt unter dem Namen Galinstan bekannt ist. Galinstan ist eine eutektische Legierung, welche etwa 68,5 Gewichtsprozent Gallium sowie etwa 21,5 Gewichtsprozent Indium und etwa 10 Gewichtsprozent Zinn aufweist. Eine solche Legierung weist einen sehr niedrigen Schmelzpunkt von etwa -19 °C auf. Andere geeignete niedrigschmelzende Legierungen sind beispielsweise unter den Namen Indalloy 51 und Indalloy 60 von der US-amerikanischen Firma Indium Corporation in Utica, NY erhältlich. Weitere geeignete galliumbasierte Legierungen sind beispielsweise diejenigen Legierungen, die in den Patentschriften US5800060B1 und US7726972B1 beschrieben sind. Sie können neben den drei genannten besonders bevorzugten Metallen auch Zusätze von anderen Metallen wie beispielsweise Zink (insbesondere zwischen etwa 2 und 10 Gewichtsprozent) umfassen. Die beschriebenen Legierungen, welche auf Gallium, Indium und/oder Zinn basieren, haben den Vorteil, dass sie eine geringe Toxizität aufweisen und somit relativ unbedenklich bezüglich Gesundheits- und Umweltschädigungen sind.

Allgemein sind Flüssigmetalle im Wesentlichen unkomprimierbar, so dass das Volumen des flüssigen Widerstandselements bei Änderungen des radialen Drucks im Wesentlichen konstant bleibt. So führt eine Erhöhung des radialen Drucks sowohl zu einer Verringerung der Querschnittsfläche A des verformbaren länglichen Widerstandselements als auch zu einer entsprechenden Erhöhung seiner Länge l, welche nötig ist, um das Flüssigkeitsvolumen konstant zu halten. Beide Veränderungen bewirken eine Erhöhung des zu messenden Längswiderstands, so dass die Empfindlichkeit der Messung bei wenig komprimierbaren deformierbaren Leitermaterialien allgemein besonders groß ist. Da auch andere Flüssigkeiten eine geringe Kompressibilität aufweisen, ist die Verwendung von flüssigen oder pastenförmigen Leitermaterialien für das verformbare Widerstandselement im Zusammenhang mit dieser Erfindung besonders zweckmäßig. Alternativ zu einer leitfähigen Flüssigkeit kann daher auch eine leitfähige Paste, insbesondere eine Paste mit leitfähigen metallischen Partikeln als verformbares Leitermaterial zum Einsatz kommen.

Gemäß einer weiteren alternativen Ausführungsform kann das reversibel verformbare Widerstandselement ein leitfähiges Elastomer sein, beispielsweise ein metallgefülltes leitfähiges Silikon. Ein leitfähiges Elastomer weist zwar eine höhere Kompressibilität auf als ein flüssiger oder pastenförmiger Leiter. Doch auch hier kann durch eine Änderung des radialen Drucks eine Änderung der Querschnittsfläche bewirkt werden, welche zu einer messbaren Veränderung des Längswiderstands führt und somit eine Messung der am Zugseil anliegenden Zugspannung ermöglicht.

Allgemein und unabhängig von der konkreten Wahl für das Material des verformbaren Widerstandselements kann das Sensorelement eine verformbare Sensorhülse aufweisen, wobei diese verformbare Sensorhülse wenigstens einen innenliegenden Kanal aufweist, in dem das verformbare längliche Widerstandselement angeordnet ist. Die Sensorhülse kann insbesondere elastisch verformbar sein. Eine solche elastisch verformbare Sensorhülse kann beispielsweise aus einem Silikon oder aus einem Gummi gebildet sein. Der wenigstens eine innenliegende Kanal kann zylinderförmig und insbesondere kreiszylindrisch ausgebildet sein, und im Inneren das Kanals kann ein entsprechend zylindrisch geformtes längliches Widerstandselement angeordnet sein. Auf diese Weise kann die Sensorhülse das innenliegende deformierbare Widerstandselement gegen die äußere Umgebung kapseln und es somit vor Umgebungseinflüssen schützen. Außerdem kann die Sensorhülse die vom Seilmantel aus einwirkende radiale Druckkraft in geeigneter Weise auf das verformbare Wider-

standelement weiterleiten und verteilen. Insbesondere kann die äußere Kontur der Sensorhülse so ausgebildet sein, dass eine relativ gleichmäßige Verteilung der Kräfte auf das innenliegende Widerstandselement erreicht wird.

Die Sensorhülse kann vorteilhaft wenigstens ein deformierbares Rohr aufweisen, welches das verformbare längliche Widerstandselement umgibt. Auch dieses deformierbare Rohr kann insbesondere elastisch verformbar sein. Ein solches Rohr kann nach radial außen hin eine Kapselung für das verformbare Widerstandselement bilden. Dies ist vor allem dann zweckmäßig, wenn das Widerstandselement aus einem flüssigen oder pastenförmigen Leitermaterial gebildet ist. Auch ein solches innenliegendes Rohr kann beispielsweise aus Silikon gebildet sein.

Gemäß einer vorteilhaften Weiterbildung der Erfindung kann das Sensorelement im Inneren der Sensorhülse auch mehrere deformierbare Rohre aufweisen, welche jeweils ein zugeordnetes verformbares längliches Widerstandselement umgeben. Auf diese Weise kann beispielsweise die Empfindlichkeit der Messung erhöht werden, indem mehrere Widerstandselemente genutzt werden, um ein von der radialen Druckkraft abhängiges elektrisches Signal zu erzeugen. Hierzu können die Widerstandselemente beispielsweise untereinander elektrisch parallelgeschaltet sein. Alternativ können die einzelnen Widerstandselemente auch elektrisch in Serie geschaltet sein, oder aber es kann eine Kombination aus Parallel- und Serienschaltung vorliegen.

Alternativ zu der Ausführungsform mit einem oder mehreren innenliegenden Rohren kann der wenigstens eine innenliegende Kanal aber auch durch eine Ausnehmung aus dem Material der Sensorhülse gebildet sein. Bei dieser "rohrlosen" Variante wird entsprechend der innenliegende Kanal, in dem das verformbare Widerstandselement angeordnet ist, von dem Material der Sensorhülse selbst begrenzt. So kann zum Beispiel durch eine Sensorhülse aus Silikon oder Gummi auch ohne innenliegendes Rohr eine hinreichende Kapselung bzw. ein hinreichender Schutz des verformbaren Widerstandselements erreicht werden.

Allgemein und unabhängig davon, wie der wenigstens eine Kanal gebildet ist und durch welches Material er begrenzt ist, kann ein solcher Kanal in seinen beiden axialen Endbereichen durch elektrisch leitende Kontaktstopfen begrenzt sein. Ein solcher Kontaktstopfen erfüllt eine doppelte Funktion: Einerseits dient er dazu, den innenliegenden Kanal für das Material des Widerstandselements nach außen abzudichten. Dies ist vor allem bei flüssigen oder pastenförmigen Leitermaterialen besonders wichtig. Andererseits dient ein solcher Kontaktstopfen aber auch dazu, das in dem Kanal angeordnete Widerstandselement im axialen Endbereich elektrisch zu kontaktieren, um so die elektrische Messung des Längswiderstands zu ermöglichen. Als Material für diese leitfähigen Kontaktstopfen eignet sich beispielsweise Kupfer oder eine Kupferlegierung. Es kommt aber auch zum Beispiel ein leitfähiger Kleber in Betracht, mit dem der Kanal besonders gut fluiddicht abgedichtet werden kann, um ein flüssiges oder pastenförmiges Leitermaterial einzukapseln.

Alternativ zu der Variante mit zwei solchen leitfähigen Kontaktstopfen kann es aber auch ausreichend sein, wenn nur an einem axialen Ende des Kanals ein solcher Kontaktstopfen vorliegt, und der Kanal am gegenüberliegenden Ende auf andere Weise elektrisch abgedichtet und kontaktiert ist. So kann der Kanal beispielsweise nur einseitig offen sein, so dass er am gegenüberliegenden Ende durch das Material der Sensorhülse selbst begrenzt ist. Wenn die Sensorhülse aus einem Vergussmaterial hergestellt ist, kann an diesem Ende ein elektrisch leitfähiges Kontaktelement zur Kontaktierung des länglichen Widerstandselements mit eingegossen sein, welches durch eine ebenfalls mit eingegossene Leitung nach außen hin kontaktiert ist.

Gemäß einer weiteren vorteilhaften Weiterbildung der Ausführungsform mit der Sensorhülse kann in dem wenigstens einen innenliegenden Kanal zwischen den beiden axialen Endbereichen eine zusätzliche Leiterfaser geführt sein. Beispielsweise kann eine solche zusätzliche Leiterfaser aus einem dünnen metallischen Draht gebildet sein, welcher innerhalb des Kanals verläuft. Der Längswiderstand dieses Drahts kann vorteilhaft groß sein im Vergleich zum Längswiderstand des übrigen deformierbaren Widerstandselements, welches ebenfalls in dem Kanal angeordnet ist. Dann wird der Wert des insgesamt gemessenen Längswiderstands im normalen Betriebszustand nur geringfügig von dieser zusätzlichen Leiterfaser beeinflusst. Allerdings verhindert eine solche zusätzliche Leiterfaser ein vollständiges Abreißen der elektrischen Leitfähigkeit in Längsrichtung. Dies ist vor allem dann relevant, wenn das übrige Leitermaterial des länglichen Widerstandselements ein flüssiges oder pastenförmiges Material ist, bei dem es bei einer starken Deformation des Kanals ansonsten zu einem solchen Abriss kommen könnte. Die Leiterfaser kann daher zweckmäßig über die Kontaktstopfen oder sonstige in den axialen Endbereichen vorliegende Kontaktelemente mit nach außen führenden Anschlussleitungen verbunden sein.

Allgemein und unabhängig von der genauen Ausgestaltung des Widerstandselements ist es vorteilhaft, wenn die Außenkontur des Sensorelements auf einem überwiegenden Teil seiner axialen Länge konvex geformt ist, so dass durch die axiale Zugkraft auf dem Zugseil ein radialer Druck auf das Sensorelement bewirkt werden kann. Durch diese Formgebung wird also nicht nur lokal (z.B. in den axialen Endbereichen), sondern auf einem Großteil der Länge des Sensorelements die Entstehung einer radialen Druckkraft aufgrund der Zugkraft auf dem Seilmantel bewirkt. Die Formgebung ist dabei vorteilhaft so, dass der entstehende radiale Druck über die überwiegende Länge des Sensorelements möglichst einheitlich ist. Dies wird durch eine sich möglichst weit erstreckende konvexe Form erreicht. In den axialen Endbereiche kann die konvexe Form des Sensorelements allerdings optional in spitze Endstücke übergehen, um eine Zentrierung des Sensorelements innerhalb des Seilmantels zu fördern.

Die beschriebene günstige Form des Sensorelements muss dabei nicht zwingend durch eine konvexe Außenkontur des Widerstandselements erreicht werden. Vielmehr kann das Widerstandselement und gegebenenfalls das umgebende deformierbare Rohr zur einfacheren Herstellung zylindrisch geformt sein, wobei die gewünschte Außenkontur dann durch eine entsprechend geformte umgebende Sensorhülse oder auch eine zusätzliche Anpassungshülse erreicht wird. Optional kann diese Sensorhülse oder Anpassungshülse durch Ausnehmungen in axial beabstandete Rippen strukturiert sein, um innerhalb der einzelnen Rippen eine möglichst radial ausgerichtete lokale Kraftkomponente auf das Widerstandselement zu bewirken. Die Zwischenräume zwischen diesen Rippen können wiederum zur Schonung des außenliegenden Seilmantels mit einem elastischen Material gefüllt sein. Die Formgebung einer solchen äußeren Hülse ist in der noch nicht offengelegten deutschen Patentanmeldung mit dem Anmeldeaktenzeichen 10 2020 213 745.5 näher beschrieben, so dass diese Anmeldung vor allem in Bezug auf die vorteilhaften Merkmale der Außenkontur in den Offenbarungsgehalt der vorliegenden Anmeldung mit einbezogen sein soll.

Alternativ zu der Ausführungsform mit einer konvexen Außenkontur kann jedoch die Außenkontur des Sensorelements (und insbesondere einer außenliegenden Sensorhülse) auch allgemein zylindrisch und insbesondere kreiszylindrisch geformt sein.

Allgemein vorteilhaft kann das Zugseil zwei elektrische Anschlussleitungen zur Verbindung des Widerstandselements mit einer Auswerteeinheit aufweisen. Diese elektrischen Anschlussleitungen sind vorteilhaft in den beiden axialen Endbereichen mit dem verformbaren Widerstandselement leitend verbunden, beispielsweise über die beschriebenen Kontaktstopfen oder anderweitige axial endständige Kontaktelemente. Die beiden Anschlussleitungen können im Bereich des Sensorelements parallel zur Seilachse geführt sein. Die Leitungen können entweder an demselben axialen Ende des betreffenden Teilabschnitts oder auch an gegenüberliegenden axialen Enden aus dem Bereich des Sensorelements ausgeleitet sein. Im ersten Fall ergibt sich eine besonders einfache gemeinsame Leitungsführung zu der Auswerteelektronik, welche entweder im Bereich des Zugseils oder außerhalb liegen kann. Die Leitungen müssen dann gegebenenfalls elektrisch gegeneinander isoliert werden, wenn sie eng beieinander geführt sind. Allgemein können die Anschlussleitungen beispielsweise als Drähte, Litzen oder Kabel realisiert sein. Sie können durch den Seilmantel hindurchgeführt sein, indem sie im Anschlussbereich durch das Geflecht der einzelnen Seilstränge bzw. Fasern radial nach außen hindurchtreten. Wenn die beiden Leitungen am selben Ende herausgeführt sind, dann kann eine der beiden Leitungen in das Material der Sensorhülse eingebettet sein, um an dem Widerstandselement vorbei zum gegenüberliegenden Ende herausgeleitet zu werden.

Der Seilmantel kann allgemein bevorzugt zumindest überwiegend aus einem synthetischen Kunststoff bestehen. Bei dem synthetischen Kunststoff kann es sich vorteilhaft um ein Polyethylen mit ultrahoher Molekülmasse (insbesondere Dyneema oder Spectra), um einen aromatischen Polyester (insbesondere Vectran), um ein Aramid (insbesondere Kevlar oder Nomex), ein Poly(p-phenylen-2,6-benzobisoxazol) (insbesondere PBO oder Zylon) oder ein Polyamid handeln. Diese Materialien zeichnen sich durch eine besonders hohe Zugfestigkeit aus. Außerdem sind sie flexibel und elektrisch nicht leitfähig, was für viele Anwendungen ebenfalls vorteilhaft ist. Allgemein vorteilhaft und unabhängig von der genauen Materialwahl kann die spezifische Zugfestigkeit des Materials des Seilmantels beispielsweise bei 2500 $N/mm^2$ oder höher liegen. Eine derart hohe Zugfestigkeit erlaubt es, Zugseile mit geringen Seildurchmessern für die Übertragung von hohen Zugkräften zu verwenden. Beispielsweise kann der Seildurchmesser in den Bereichen außerhalb des von dem Sensorelement besetzten Teilbereichs zwischen 0,5 mm und 10 mm liegen. Dagegen kann der Seildurchmesser in dem mit dem Sensorelement gefüllten Teilbereich beispielsweise um einen Faktor 3 vergrößert sein. So kann beispielsweise ein Seilmantel mit einem regulären Außendurchmesser von 4 mm im Bereich des Sensorelements einen vergrößerten Außendurchmesser von bis zu 12 mm aufweisen. Allgemein kann der Faktor für den Seildurchmesser vorteilhaft in einem Bereich zwischen 1,5 und 5 liegen. Bei einem vergleichsweise hohen Faktor ist die Empfindlichkeit der Zugspannungsmessung besonders hoch, allerdings steigt auch die mechanische Belastung des Seilmantels im Bereich des Sensorelements.

Die Erfindung ist nicht auf Seilmäntel aus hochfesten synthetischen Kunststoffen beschränkt. So kann der Seilmantel alternativ auch aus herkömmlichen Materialien, beispielweise aus Stahl, Kupfer oder Hanf bestehen und in entsprechender Weise lokal mit einem Sensorelement zur Zugkraftmessung gefüllt sein.

Allgemein kann das Zugseil aus mehreren Teilseilen bestehen, welche jeweils einen separaten Seilmantel aufweisen. In einem solchen Fall ist es ausreichend, wenn zumindest eines dieser Teilseile zur Zugkraftmessung lokal mit einem innenliegenden Sensorelement versehen ist.

Gemäß einer vorteilhaften Ausführungsform der seilbetriebenen Einrichtung ist der Teilabschnitt mit dem innenliegenden Sensorelement in einem frei verlaufenden Bereich des Zugseils angeordnet. Ein solcher frei verlaufender Bereich ist ein Abschnitt des Zugseils, in dem kein mechanischer Kontakt zu einer Umlenkrolle vorliegt, keine Befestigungspunkte vorliegen und das Zugseil auch sonst nicht an anderen führenden Elementen mechanisch anliegt.

Die seilbetriebene Einrichtung kann eine Auswerteeinheit umfassen, mit welcher die von der Zugkraft abhängige Änderung des Längswiderstands gemessen werden kann. Diese Auswerteeinheit kann entweder als Teil des Zugseils oder separat dazu ausgeführt sein. Die Auswerteeinheit kann beispielsweise eine Wheatstone-Brückenschaltung umfassen.

Die seilbetriebene Einrichtung kann beispielsweise als Robotikeinrichtung ausgestaltet sein. So kann das Zugseil innerhalb der Robotikeinrichtung zur Übertragung einer Zugkraft dienen, beispielsweise um eine robotische Gelenkeinheit mit Hilfe einer integrierten Antriebsvorrichtung zu bewegen. Das Zugseil kann mit anderen Worten eine Funktion analog zu einer Sehne im menschlichen Körper übernehmen. Alternativ zu einer "Antriebssehne" (welche Antriebseinheit und Abtriebselement kraftübertragend verbindet) kann eine solche Sehne aber auch die Funktion einer Stützverspannung erfüllen, durch welche die beweglichen Elemente des Antriebsstrangs gegen die mechanische Masse oder andere mechanisch starre Elemente abgestützt werden. Beispielsweise kann das erfindungsgemäße Zugseil in eine Stützverspannung einer sogenannten Tensegrity-Struktur integriert sein.

Gemäß einer besonders vorteilhaften Ausführungsform des Messverfahrens kann dieses dazu ausgelegt sein, auch schnelle Änderungen der Zugspannung durch Messung des elektrischen Signals zu erfassen. Auf diese Weise können Schwingungen im Bereich des Zugseils gemessen werden. Besonders vorteilhaft kann eine solche Messung als Grundlage einer aktiven Schwingungsdämpfung in einer seilbetriebenen Einrichtung genutzt werden.

Gemäß einer vorteilhaften Weiterbildung des Verfahrens erfolgt die Messung des vom Längswiderstand beeinflussten elektrischen Signals mittels einer Wheatstone-Brückenschaltung. Auf diese Weise kann eine besonders präzise und empfindliche Messung der Widerstandsänderung und somit der zugrundeliegenden Änderung in der Zugspannung des Seils erfolgen. Vorteilhaft kann es sich dabei um ein selbstkompensiertes System handeln, bei dem der Messwiderstand (oder zumindest das darin enthaltene verformbare Widerstandselement) einen negativen Temperaturkoeffizienten aufweist. Auf diese Weise kann durch die Temperaturabhängigkeit der spezifischen Leitfähigkeit eine automatische Kompensation einer mit einer Temperaturänderung einhergehenden geometrischen Veränderung des Widerstandselements erfolgen. Auf diese Weise kann eine temperaturbedingte geometrische Änderung, welche nicht durch eine Änderung der Zugspannung verursacht ist und entsprechend nicht zu einer Signaländerung führen soll, bei der Messung zumindest teilweise kompensiert werden.

Alternativ oder zusätzlich kann es vorteilhaft sein, wenn die Wheatstone-Brückenschaltung einen zweiten aktiven Messwiderstand aufweist, welcher einen ähnlichen Temperaturverlauf wie der erste Messwiderstand erfährt. Der zweite Messwiderstand kann insbesondere ein längliches Widerstandselement aus demselben Material aufweisen wie der erste Messwiderstand (also z.B. ein Flüssigmetall) und er kann innerhalb der seilbetriebenen Einrichtung in der Nähe des ersten Messwiderstands angeordnet sein, so dass er vergleichbare Temperaturänderungen erfährt. Zweckmäßig ist er aber nicht derselben Verformung aufgrund der veränderten Zugspannung ausgesetzt, so dass durch diesen zweiten Messwiderstand eine automatische Kompensation für die Temperaturabhängigkeit des Widerstandsmaterials erfolgen kann.

Im Rahmen der Erfindung ergeben sich auch besonders vorteilhafte Ausgestaltungen für das Herstellungsverfahren des erfindungsgemäßen Zugseils und insbesondere des darin enthaltenen länglichen Sensorelements. So kann das Sensorelement vorteilhaft als vorgefertigtes Bauteil zerstörungsfrei in das Innere des vorgefertigten Seilmantels eingeführt werden, indem der Seilmantel vor diesem Verfahrensschritt temporär aufgeweitet wird, wodurch sich die Zwischenräume des Geflechts temporär vergrößern. Nach dem Einschieben können die einzelnen Seilstränge wieder zu einem symmetrischen Geflecht zusammengeschoben werden.

Das vorgefertigte Sensorelement kann vorteilhaft dadurch erzeugt werden, dass zunächst eine Sensorhülse mit einer passenden Ausnehmung für den wenigstens einen innenliegenden Kanal für das Widerstandselement hergestellt wird. Der jeweilige innenliegende Kanal kann dann optional mit einem deformierbaren Rohr ausgekleidet werden und jedenfalls mit dem Material des deformierbaren Widerstandselements befüllt werden. Der Kanal kann anschließend mit optionalen endständigen Kontaktstopfen oder auch auf andere Weise gegen die äußere Umgebung verschlossen werden. Alternativ kann aber auch das jeweilige Rohr mit dem darin enthaltenen Widerstandselement als vorbefülltes Rohr in die Vergussform eingeführt und dann von der Vergussmasse der Sensorhülse umgossen werden. Dabei können optional auch ein oder mehrere Kontaktelemente und/oder zugehörige Anschlussleitungen mit eingegossen werden, was zu einer verbesserten Dichtigkeit gegenüber einem flüssigen Leitermaterial beitragen kann.

Die Sensorhülse kann vorteilhaft durch ein Vergussverfahren hergestellt werden. Hierzu kann eine Vergussform verwendet werden, welche mittels additiver Fertigung hergestellt ist.

In jedem Fall kann mittels einer entsprechend geformten Vergussform auch die vorteilhafte konvexe Außenkontur erzeugt werden. Alternativ kann auch die Sensorhülse selbst mit ihrem wenigstens einen innenliegenden Kanal und der jeweils gewünschten Außenkontur mittels additiver Fertigung hergestellt sein. So können auch komplexer geformte innenliegende Hohlräume für ein oder mehrere deformierbare Widerstandselemente realisiert werden.

Nachfolgend wird die Erfindung anhand einiger bevorzugter Ausführungsbeispiele unter Bezugnahme auf die angehängten Zeichnungen beschrieben, in denen:

| | |
|---|---|
| Figur 1 | eine schematische Aufsicht eines Zugseils zeigt, in das ein Sensorelement eingeführt wird, |
| Figur 2 | ein Zugseil mit einem darin eingeführten Sensorelement zeigt, |
| Figuren 3 und 4 | schematische perspektivische Darstellungen von Sensorelementen zeigen, |
| Figur 5 | einen schematischen perspektivischen Teilschnitt eines Sensorelements in einem Zugseil zeigt, |
| Figur 6 | eine Schnittdarstellung eines weiteren Sensorelements zeigt, |
| Figur 7 | eine Vergussform für die Herstellung eines Sensorelements zeigt und |
| Figur 8 | eine schematische Darstellung einer WheatstoneBrückenschaltung zeigt. |

In den Figuren sind gleiche oder gleichwirkende Elemente mit gleichen Bezugszeichen versehen.

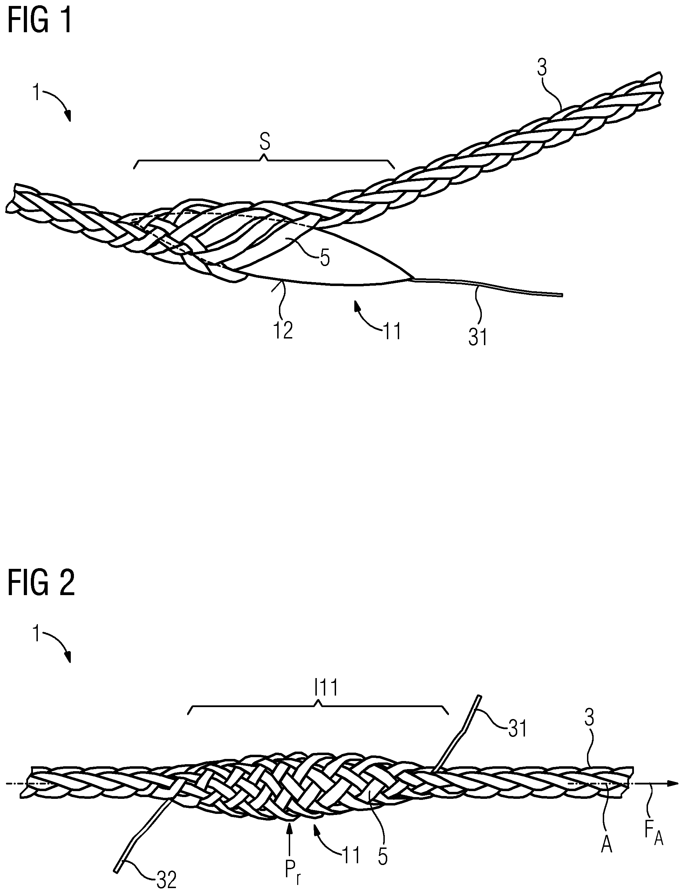

In Figur 1 ist eine schematische Aufsicht eines Zugseils 1 nach einem ersten Beispiel der Erfindung gezeigt, und zwar während eines Herstellungsschrittes, bei dem ein Sensorelement 11 in das Innere des Seilmantels 3 des Zugseils eingeführt wird. Der Seilmantel 3 ist ein schlauchartiges Geflecht aus mehreren Seilsträngen bzw. Fasern, von denen eine exemplarisch mit 5 bezeichnet ist. Diese einzelnen Stränge bzw.

Fasern liegen dabei jeweils schräg zur lokalen Seilachse. Durch Aufstauchen des Seilmantels 3 und temporäres Vergrößern der Zwischenräume des Geflechts kann das Sensorelement zerstörungsfrei in das Innere des Seilmantels 3 eingeführt werden. In Figur 2 ist der Zustand des Zugseils 1 zu sehen, bei dem das Sensorelement 11 in das Innere des Seilmantels eingeführt ist und die einzelnen Seilstränge 5 wieder zu einem symmetrischen Geflecht zusammengeschoben sind. Die beiden Figuren illustrieren also, wie ein erfindungsgemäßes Zugseil auf einfache Weise durch nachträgliche Modifikation eines herkömmlichen Seils erzeugt werden kann, welches einen schlauchartigen Seilmantel mit einem innenliegenden Hohlraum aufweist. Das Sensorelement wird dabei nur in einen Teilabschnitt des Zugseils 1 eingeführt, der der Länge l11 des Sensorelements entspricht und in welchem der Durchmesser anschließend entsprechend vergrößert ist. In den übrigen Abschnitten bleibt das Zugseil dagegen unverändert. Insgesamt werden die wesentlichen Eigenschaften wie Zugfestigkeit und axiale Steifigkeit durch den Einschub des Sensorelements nur unwesentlich verändert.

Das Sensorelement 11 ist länglich geformt und liegt im fertig eingeschobenen Zustand koaxial zur Seilachse A. Es weist in diesem Beispiel eine Außenkontur 12 auf, welche über einen wesentlichen Teil seiner Länge konvex geformt ist. Trotzdem läuft sie in den axialen Endbereichen spitz zu. Dieses spitze Zulaufen erleichtert eine Zentrierung des Sensorelements im Seilmantel unter Zugbelastung. Bei Anliegen einer Zugkraft $F_A$ auf dem Seilmantel 3 resultiert aufgrund der Schräglage der umgebenden Seilfasern 5 eine radiale Druckkraft $p_r$ auf das innenliegende Sensorelement. Aufgrund der konvexen Außenkontur 12 des Sensorelements weist die resultierende Druckkraft über einen großen Teil der Länge des Sensorelements eine hohe radiale Komponente auf, welche mit Hilfe des Sensorelements gemessen werden kann, um so ein Maß für die Größe der anliegenden Zugkraft $F_A$ zu erhalten. Die Messung erfolgt über die Messung eines elektrischen Signals, welche durch ein deformierbares Widerstandselement im Inneren des Sensorelements beeinflusst wird. Um diese elektrische Messung zu ermöglichen, weist das Sensorelement zwei Anschlussleitungen 31 und 32 auf. Diese verlaufen innerhalb des Seilmantels 3 zunächst parallel zur Seilachse A, werden dann aber durch schrägliegende Endabschnitte durch das Seilgeflecht des Mantels aus dem Inneren herausgeführt. So kann eine elektrische Messung über eine hier nicht näher dargestellte Auswerteeinheit ermöglicht werden. Alternativ zu der hier dargestellten Kontur kann das Sensorelement 11 aber auch eine einfachere und insbesondere zylindrische Außenkontur aufweisen, wobei die Verteilung des radialen Drucks dann nicht ganz so gleichmäßig ist, das zugrundeliegende Messprinzip aber trotzdem analog ist.

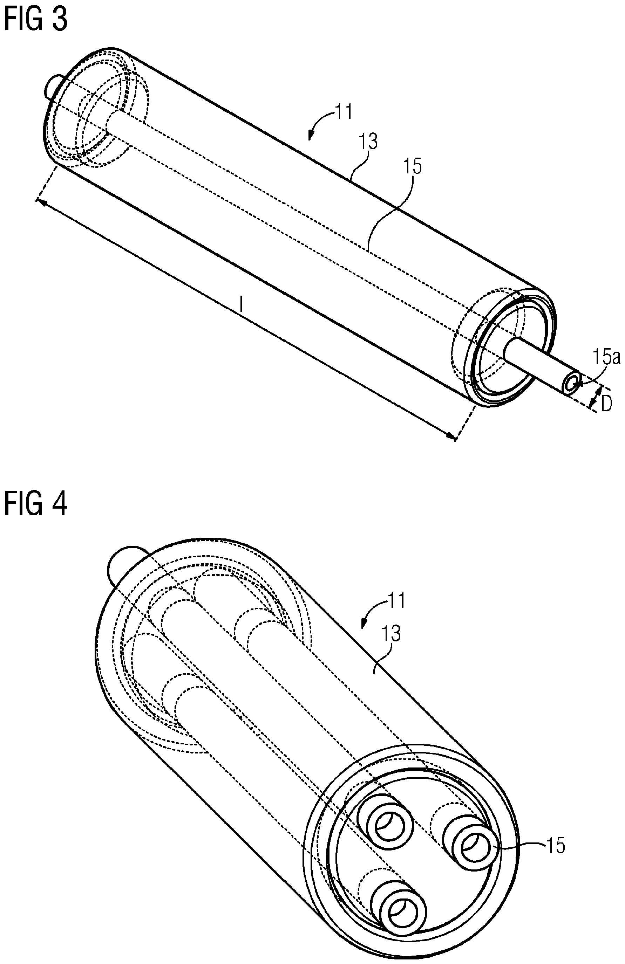

So zeigt Figur 3 eine schematische perspektivische Darstellung eines Sensorelements 11 mit kreiszylindrischer Außenkontur nach einem ersten Beispiel der Erfindung. Dieses Sensorelement kann analog wie das Sensorelement der Figuren 1 und 2 im Inneren eines Zugseils angeordnet sein und dort zur Messung der Zugkraft $F_A$ dienen. Das Sensorelement 11 der Figur 3 weist eine Sensorhülse 13 mit kreiszylindrischer Grundform auf. Diese Sensorhülse ist aus einem verformbaren Material gebildet, beispielsweise aus einem elastisch verformbaren Silikon, so dass sie von außen einwirkende Druckkräfte an das Innere der Sensorhülse weiterleiten kann. Die Sensorhülse 13 weist in ihrem Inneren einen länglichen Kanal auf, in dem bei diesem Beispiel ein innenliegendes Rohr 15 angeordnet ist. Auch dieses Rohr 15 ist aus einem verformbaren Material gebildet, beispielsweise aus elastisch verformbarem Silikon, so dass auch die Rohrwand die einwirkenden Druckkräfte an das Innere des Rohrs weiterleiten kann. Im Inneren des Rohrs ist ein hier nicht näher dargestelltes reversibel verformbares Widerstandselement angeordnet, beispielsweise eine Füllung aus Flüssigmetall. Dieses Widerstandselement kann durch eine Öffnung 15a in das Innere des Rohrs eingefüllt werden. Es weist eine Länge l und einen Durchmesser D auf, wobei diese beiden geometrischen Parameter unter dem Einfluss radialer Druckkräfte variieren können. Die Änderung dieser geometrischen Parameter kann durch eine elektrische Messung verfolgt werden, nämlich zweckmäßiger Weise durch eine Messung des Längswiderstands des innenliegenden Widerstandselements. Hierzu kann das Rohr 15 in seinen axialen Endbereichen mit hier nicht näher darstellten Kontaktstopfen verschlossen sein, wobei die Kontaktstopfen eine elektrische Verbindung zu ebenfalls nicht gezeigten Anschlussleitungen für die elektrische Messung ausbilden. Die effektive Länge l des Widerstandselements ergibt sich dann aus dem Längenabschnitt zwischen den Kontaktstopfen. Der Längswiderstand R des dazwischenliegenden Widerstandselements ergibt sich damit als

$$R = (\rho * l)/(\pi * D^2/4),$$

wobei ρ der spezifische Widerstand des verformbaren Leitermaterials ist. Eine erhöhte Zugspannung auf dem Seil führt zu einem erhöhten radialen Druck und somit zu einem radialen Zusammenpressen des verformbaren Widerstandselements. Dies führt sowohl zu einer Verringerung des Durchmessers D als auch zu einer Erhöhung der Länge l. Beide Effekte bewirken eine Erhöhung des gemessenen Längswiderstands. In ähnlicher Weise führt eine Verringerung der Zugspannung zu einer Erniedrigung des gemessenen Längswiderstands. Dieses allgemeine Prinzip der Widerstandsänderung durch Verformung ist auf alle länglich geformten deformierbaren Leiterelemente (auch mit nicht kreiszylindrischen Geometrien) übertragbar.

Figur 4 zeigt eine schematische perspektivische Darstellung eines Sensorelements 11 nach einem zweiten Beispiel der Erfindung, welches ebenfalls im Inneren eines Zugseils zur Messung der Zugspannung zum Einsatz kommen kann. Ähnlich wie beim Beispiel der Figur 3 liegt auch hier eine längliche Sensorhülse 13 aus einem verformbaren Material mit einem kreiszylindrischen Querschnitt vor. Im Unterschied zum vorhergehenden Beispiel weist diese Sensorhülse aber nicht nur einen, sondern drei innenliegende Kanäle auf. Auch in diesen Kanälen ist jeweils ein verformbares Rohr 15 angeordnet, welches jeweils mit einem verformbaren Widerstandselement befüllt ist.

Diese drei Rohre können exemplarisch auch für eine noch deutlich höhere Anzahl von Rohren stehen, oder es können auch nur zwei Rohre im Inneren der Sensorhülse vorliegen. In jedem Fall kann die Mehrzahl von enthaltenen Widerstandselementen zu einer Erhöhung der Empfindlichkeit und/oder Genauigkeit der Messung beitragen. Hierzu können die einzelnen Widerstandselemente durch hier nicht dargestellte Anschlussleitungen untereinander und mit einer übergeordneten Auswerteeinheit elektrisch verbunden sein.

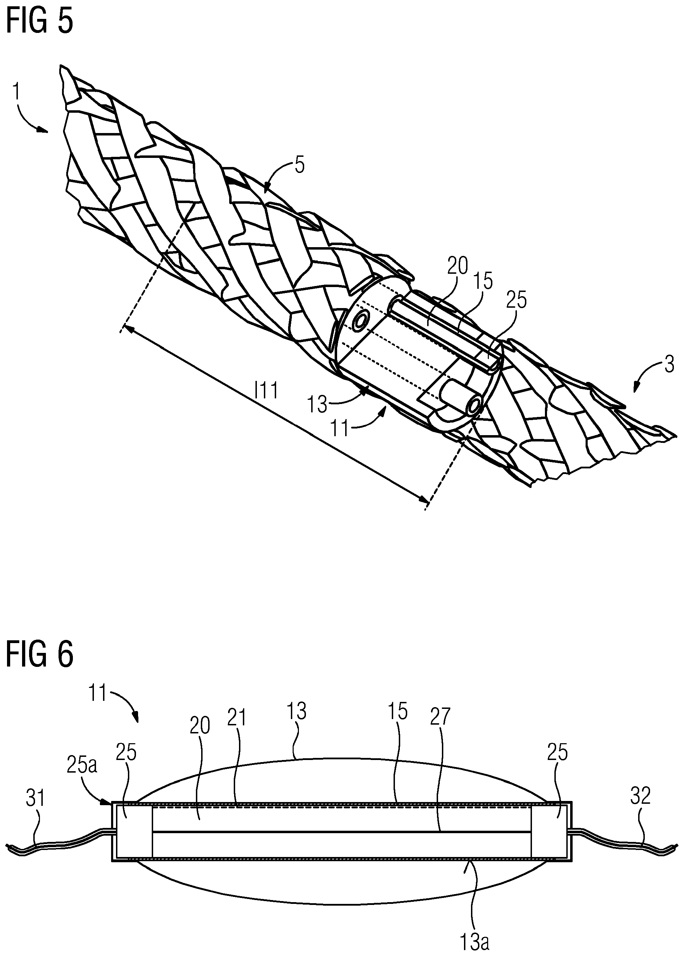

In Figur 5 ist eine schematische perspektivische Schnittansicht des Sensorelements 11 der Figur 4 im Inneren eines Zugseils 1 gezeigt. Ähnlich wie beim Beispiel der Figur 2 ist auch hier das Sensorelement 11 in das Innere eines Seilmantels 3 eingeschoben worden. Die Änderung der vom Seilmantel 3 ausgeübten radialen Druckkraft kann durch Änderungen der Längswiderstände der innenliegenden Widerstandselemente 20 gemessen werden. Hierzu können die einzelnen Widerstandselemente elektrisch zueinander parallelgeschalter oder in Serie geschaltet sein oder sie können auch einzeln mit einer übergeordneten Auswerteeinheit verbunden sein.

Figur 6 zeigt ein Sensorelement 11 nach einem weiteren Beispiel der Erfindung im schematischen Längsschnitt. Auch hier weist das Sensorelement eine außenliegende Sensorhülse 13 auf, deren Form die Außenkontur des Sensorelements bestimmt. Im Unterschied zu den vorhergehenden Beispielen ist die Sensorhülse hier konvex geformt, so dass die Zugspannung eines umgebenden Seilmantels auf der gesamten Länge des Sensorelements zu einer relativ gleichmäßig verteilten radialen Druckkraft führt, ähnlich wie beim Beispiel der Figur 2 mit ebenfalls konvexer Außenkontur. Beim Beispiel der Figur 6 ist nur die Außenseite der Sensorhülse 13 konvex geformt. Ihr innenliegender Kanal ist wiederum kreiszylindrisch geformt, und auch hier ist in diesem Kanal ein kreiszylindrisches Rohr 15 angeordnet, ähnlich wie beim Beispiel der Figur 3. Auch hier ist das Rohr mit einem deformierbaren Leiterelement 20 befüllt. Die beiden axialen Endbereiche des Rohrs 15 sind nach außen hin durch elektrisch leitende Kontaktstopfen 25 verschlossen, so dass kein Leitermaterial nach außen entweichen kann. Diese Kontaktstopfen können beispielsweise aus Abschnitten von Kupferdrähten gebildet sein. Sie sind mit elektrischen Anschlussleitungen 31 und 32 zur Durchführung der elektrischen Messung verbunden. Um elektrische Kurzschlüsse zu vermeiden, sind die Kontaktstopfen 25 auf ihrer Außenseite mit einer elektrischen Isolationsschicht 25a versehen.

Beim Beispiel der Figur 6 ist das verformbare Leitermaterial 20 durch ein Flüssigmetall gegeben, beispielsweise Galinstan. Beim Befüllen des Rohrs 15 mit diesem Flüssigmetall kann ein Lufteinschluss 21 im Rohr verbleiben. Aufgrund solcher Lufteinschlüsse kann es beim Betrieb des Zugseils gegebenenfalls auch zu Abrissen des Widerstandselements 20 kommen, vor allem dann, wenn der durchschnittliche Querschnitt des Widerstandselements 20 sehr gering ist. Um eine vollständige Unterbrechung der leitfähigen Verbindung zu vermeiden, kann das Sensorelement eine optionale innenliegende Leiterfaser 27 aufweisen, beispielsweise einen dünnen Kupferdraht. Der Widerstand dieser Leiterfaser kann so gewählt sein, dass er im normalen Betriebszustand (wenn also das Flüssigmetall 20 nicht abgerissen ist) deutlich größer ist als der Widerstand des Flüssigmetalls. Dann wird die Messung der geometrischen Verformung durch die innenliegende Leiterfaser nicht wesentlich beeinträchtigt. Figur 6 zeigt einen Zustand der Leiterfaser 27, in dem diese maximal gestreckt ist. Dies entspricht einem Zustand mit maximalem radialem Druck und maximaler Länge l des Widerstandselements. Wenn der radiale Druck (und somit die Zugkraft auf dem Seil) geringer ist, dann ist die innenliegende Leiterfaser nicht straff gespannt, sondern liegt gekrümmt und sozusagen mit Längenreserve im Inneren des Rohrs.

Bei den gezeigten Beispielen der Figuren 3 bis 6 ist das deformierbare Widerstandselement 20 jeweils im Inneren eines verformbaren Rohrs 15 angeordnet. Alternativ zu diesen Beispielen ist es aber prinzipiell auch möglich, dass das verformbare Widerstandselement 20 jeweils direkt in einer länglichen Ausnehmung 13a der Sensorhülse 13 angeordnet ist und nicht durch ein zusätzliches innenliegendes Rohr begrenzt ist. Es wird dann direkt durch das Material der Sensorhülse 13 nach außen hin abgedichtet. Die Geometrie ist dann analog zu den vorhergehenden Beispielen, nur ohne das jeweilige Rohr 15.

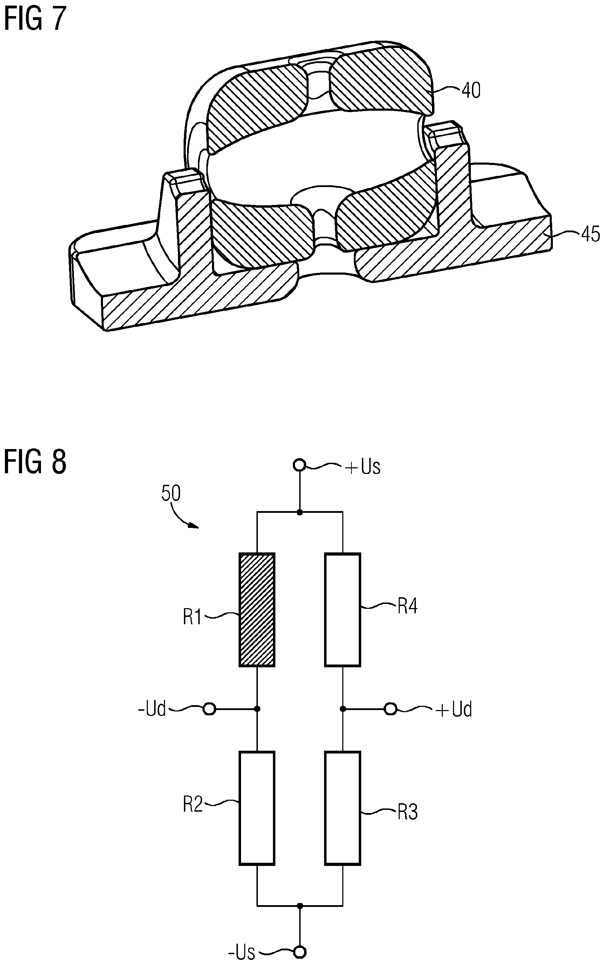

In Figur 7 ist eine Schnittdarstellung einer Vergussform 40 gezeigt, wie sie bei der Herstellung eines Sensorelements zum Einsatz kommen kann. Eine solche Vergussform 40 kann dazu verwendet werden, um eine Sensorhülse 13 für ein Sensorelement 11 aus einem Vergussmaterial zu bilden, beispielsweise aus Silikon. Hierzu kann ein Rohr 15 (mit oder ohne eingefülltes Leitermaterial 20) im Inneren der Vergussform 40 positioniert werden und dann mit dem Material der Sensorhülse umgossen werden. Die dargestellte Halterung 45 kann optional dazu eingesetzt werden, das Rohr 15 während des Vergussprozesses im Inneren der Vergussform 40 zu zentrieren. Die Vergussform 40 kann beispielsweise durch ein additives Fertigungsverfahren erzeugt worden sein.

In Figur 8 ist eine schematische Darstellung einer Wheatstone-Brückenschaltung 50 gezeigt. Diese Brückenschaltung kann Teil einer übergeordneten Auswerteeinheit sein, mit der die Änderung des Längswiderstandes des Widerstandselements 20 gemessen wird. Solche Brückenschaltungen sind aus dem Stand der Technik hinreichend bekannt. In Figur 8 ist eine Viertelbrücke dargestellt, bei der ein aktiver Messwiderstand R1 mit drei passiven Widerständen R2, R3, R4 zusammengeschaltet ist. Der Widerstand R1 entspricht dabei dem Längswiderstand eines deformierbaren Widerstandselements 20 aus einem der Beispiele, dessen zugspannungsabhängige Veränderung gemessen werden soll. Der Einfluss von zusätzlichen temperaturabhängigen Widerstandsänderungen von R1, die nicht auf einer Änderung der Zugspannung beruhen, kann dabei zumindest teilweise kompensiert werden, wenn das Material des Widerstandselements einen negativen Temperaturkoeffizienten aufweist.

Alternativ zu der vorab beschriebenen Viertelbrücke kann aber auch eine Halbbrücke zum Einsatz kommen, bei der zwei Widerstände R1 und R2 als aktive Messwiderstände ausgebildet sind und die beiden anderen R3 und R3 als passive Widerstände ausgebildet sind. Sowohl R1 als auch R2 können dann beispielsweise durch einen Flüssigmetall-Leiter gebildet sein und innerhalb der seilbetriebenen Einrichtung so angeordnet sein, dass sie ähnliche Temperaturänderungen erfahren. So können Temperatureffekte durch die Art der Messung zusätzlich kompensiert werden.

Bezugszeichenliste

| 1 | Zugseil |
|---|---|
| 3 | Seilmantel |
| 5 | Seilstränge bzw. Fasern |
| 11 | Sensorelement |
| 12 | Außenkontur |
| 13 | Sensorhülse |
| 13a | Ausnehmung der Sensorhülse |
| 15 | Rohr |
| 15a | Öffnung |
| 20 | Widerstandselement (Flüssigmetall) |
| 21 | Lufteinschluss im Rohr |
| 25 | Kontaktstopfen |
| 25a | Isolation des Kontaktstopfens |
| 27 | Leiterfaser |
| 31 | erste Anschlussleitung |
| 32 | zweite Anschlussleitung |
| 40 | Vergussform |
| 45 | Halterung |
| 50 | Wheatstone-Brückenschaltung |
| A | lokale Seilachse |
| D | Durchmesser des Widerstandselements |
| $F_A$ | axiale Zugkraft |
| l | Länge des Widerstandselements |
| l11 | Länge des Sensorelements |
| $p_r$ | radialer Druck |
| R1 | erster Messwiderstand |
| R2 | zweiter (Mess-)Widerstand |
| R3 | dritter Widerstand |
| R4 | vierter Widerstand |
| Us | Brückenspeisung |
| Ud | Brückenausgang |

**Patentansprüche**

1. Zugseil (1) zur Übertragung einer axialen Zugkraft ($F_A$), umfassend

    - einen schlauchförmigen Seilmantel (3), der ein Geflecht aus mehreren Seilsträngen und/oder Fasern (5) umfasst, wobei die einzelnen Seilstränge und/oder Fasern (5) schräg zur lokalen Seilachse (A) verlaufen, und

    - ein in einem Teilabschnitt innerhalb des Seilmantels (3) angeordnetes länglich geformtes Sensorelement (11), welches ein verformbares längliches Widerstandselement (20) aufweist, dessen elektrischer Längswiderstand von einem radialen Druck ($p_r$) des Seilmantels (3) auf das Sensorelement (11) abhängt,

    - wobei der radiale Druck ($p_r$) auf das Sensorelement (11) von der axialen Zugkraft ($F_A$) auf das Zugseil (1) abhängt.

2. Zugseil (1) nach Anspruch 1, bei dem das verformbare längliche Widerstandselement (20) ein flüssiges oder pastenförmiges Leitermaterial, insbesondere ein Flüssigmetall als Leitermaterial, aufweist.

3. Zugseil (1) nach einem der Ansprüche 1 oder 2, bei welchem das Sensorelement (11) eine elastisch verformbare Sensorhülse (13) aufweist,

   - wobei die Sensorhülse (13) wenigstens einen innenliegenden Kanal aufweist, in dem das verformbare längliche Widerstandselement (20) angeordnet ist.

4. Zugseil (1) nach Anspruch 3, bei welchem das Sensorelement (11) im Inneren der Sensorhülse (13) wenigstens ein deformierbares Rohr (15) aufweist, welches das verformbare längliche Widerstandselement (20) umgibt.

5. Zugseil (1) nach Anspruch 4, bei welchem das Sensorelement (11) im Inneren der Sensorhülse (13) mehrere deformierbare Rohre (15) aufweist, welche jeweils ein zugeordnetes verformbares längliches Widerstandselement (20) umgeben.

6. Zugseil (1) nach Anspruch 3, bei welchem der wenigstens eine innenliegende Kanal durch eine Ausnehmung (13a) aus dem Material der Sensorhülse (13) gebildet ist.

7. Zugseil (1) nach einem der Ansprüche 3 bis 6, bei welchem der innenliegende Kanal der Sensorhülse (13) in seinen beiden axialen Endbereichen durch elektrisch leitende Kontaktstopfen (25) begrenzt ist.

8. Zugseil (1) nach einem der Ansprüche 3 bis 7, bei welchem in dem innenliegenden Kanal der Sensorhülse (13) zwischen den beiden axialen Endbereichen eine zusätzliche Leiterfaser (27) geführt ist.

9. Zugseil (1) nach einem der vorhergehenden Ansprüche, bei welchem die Außenkontur (12) des Sensorelements (11) auf einem überwiegenden Teil seiner axialen Länge derart konvex geformt ist, dass durch die axiale Zugkraft ($F_A$) auf dem Zugseil (1) ein radialer Druck ($p_r$) auf das Sensorelement (11) bewirkt werden kann.

10. Zugseil (1) nach einem der vorhergehenden Ansprüche, welches zwei elektrische Anschlussleitungen (31,32) zur Verbindung der verformbaren Widerstandselements (20) mit einer Auswerteeinheit aufweist, wobei die beiden Anschlussleitungen (31,32) insbesondere parallel zur Seilachse (A) geführt sind.

11. Zugseil (1) nach einem der vorhergehenden Ansprüche, bei welchem der Seilmantel (3) zumindest überwiegend aus einem synthetischen Kunststoff besteht.

12. Seilbetriebene Einrichtung mit einer Antriebseinheit, einem Abtriebselement und einem Zugseil (1) nach einem der Ansprüche 1 bis 11, welches die Antriebseinheit und das Abtriebselement bezüglich einer Zugrichtung (A) kraftübertragend verbindet.

13. Einrichtung nach Anspruch 12, welche als Robotikeinrichtung ausgestaltet ist.

14. Verfahren zur Messung einer Zugkraft ($F_A$), welche auf ein Zugseil (1) nach einem der Ansprüche 1 bis 11 einwirkt,

   - wobei ein durch den Längswiderstand des verformbaren länglichen Widerstandselements (20) beeinflusstes elektrisches Signal gemessen wird, dessen Größe von einem radialen Druck ($p_r$) des Seilmantels (3) auf das Sensorelement (11) abhängt,
   - wobei der radiale Druck ($p_r$) auf das Sensorelement (11) von der Zugkraft ($F_A$) auf das Zugseil (1) abhängt.

15. Verfahren nach Anspruch 14, bei welchem die Messung des durch den Längswiderstand beeinflussten elektrischen Signals mittels einer Wheatstone-Brückenschaltung (50) erfolgt.

FIG 1

1

3

S

5

12

11

31

FIG 2

1

l11

31

3

32

$P_r$

11

5

A

$F_A$

FIG 3

FIG 4

## FIG 5

## FIG 6

## FIG 7

## FIG 8

Europäisches
Patentamt

European
Patent Office

Office européen
des brevets

**EUROPÄISCHER RECHERCHENBERICHT**

Nummer der Anmeldung

**EP 21 20 4272**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2020/249976 A1 (SENSOR TECH LTD [GB]) 17. Dezember 2020 (2020-12-17) | 1,3,6, 9-12,14 | INV. D07B1/14 |
| Y | * Seite 1, Zeilen 16-18; Abbildungen 1-3 * <br> * Seite 11, Zeile 9 * <br> * Seite 12, Zeilen 14-26 * <br> ----- | 2,15 | |
| X | WO 2019/076522 A1 (WILHELMSEN SHIPS SERVICE AS [NO]) 25. April 2019 (2019-04-25) | 1,3,6,9, 10,12,14 | |
| Y | * Seite 3, Zeilen 36-38; Abbildungen 2-4 * <br> * Seite 5, Zeilen 5-8 * <br> * Seite 16, Zeilen 5,6; Abbildungen 5b, 5c * <br> * Seite 18, Zeilen 19,25 * <br> * Seite 20, Zeilen 28-37; Abbildung 10 * <br> ----- | 1,14,15 | |
| Y | Anonymous: "The Strain Gage", , 4. September 2017 (2017-09-04), XP055899486, Internet Gefunden im Internet: URL:https://web.archive.org/web/2017090418 4108/https://www.omega.de/literature/trans actions/volume3/strain2.html [gefunden am 2022-03-09] * Seite 1 * ----- | 1,14,15 | |
| | | | RECHERCHIERTE SACHGEBIETE (IPC) |
| | | | G01L D07B |
| Y | CN 110 146 200 A (NINGBO INST MATERIALS TECH & ENG CAS) 20. August 2019 (2019-08-20) * Absätze [0002] - [0004], [0031]; Abbildung 2 * ----- | 2 | |
| A | DE 10 2016 210615 A1 (LEONI KABEL GMBH [DE]) 21. Dezember 2017 (2017-12-21) * Anspruch 1; Abbildungen 1,2 * ----- | 13 | |

-/--

Der vorliegende Recherchenbericht wurde für alle Patentansprüche erstellt

| Recherchenort | Abschlußdatum der Recherche | Prüfer |
|---|---|---|
| München | 5. April 2022 | Uhlig, Robert |

Europäisches
Patentamt

European
Patent Office

Office européen
des brevets

**EUROPÄISCHER RECHERCHENBERICHT**

Nummer der Anmeldung

**EP 21 20 4272**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2017/167242 A1 (KATAYAMA MAKITO [JP]) 15. Juni 2017 (2017-06-15) * Anspruch 1; Abbildung 3 * ----- | 1,14 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 5. April 2022 | Uhlig, Robert |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 20 4272

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-04-2022

| Im Recherchenbericht angeführtes Patentdokument | Datum der Veröffentlichung | Mitglied(er) der Patentfamilie | Datum der Veröffentlichung |
|---|---|---|---|
| WO 2020249976 A1 | 17-12-2020 | GB 2592148 A <br> WO 2020249976 A1 | 18-08-2021 <br> 17-12-2020 |
| WO 2019076522 A1 | 25-04-2019 | KEINE | |
| CN 110146200 A | 20-08-2019 | KEINE | |
| DE 102016210615 A1 | 21-12-2017 | CN 109073421 A <br> DE 102016210615 A1 <br> EP 3472568 A1 <br> JP 6783328 B2 <br> JP 2019518962 A <br> KR 20190008904 A <br> US 2019120706 A1 <br> WO 2017215891 A1 | 21-12-2018 <br> 21-12-2017 <br> 24-04-2019 <br> 11-11-2020 <br> 04-07-2019 <br> 25-01-2019 <br> 25-04-2019 <br> 21-12-2017 |
| US 2017167242 A1 | 15-06-2017 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5800060 B1
- US 7726972 B1
- DE 102020213745